# EUROPEAN PATENT APPLICATION

(11) **EP 1 897 888 A1**
(43) Date of publication of application: **12.03.2008**
(21) Application number: 06018604.6
(22) Date of filing: 05.09.2006
(51) Int. Cl.: C07K 14/52, A61K 38/19

(54) **Peptides binding the TPO receptor**

(71) Applicant: AplaGen GmbH, 52499 Baesweiler (DE)
(72) Inventor: Frank, Hans-Georg, 6462 EL Kerkrade (NL); Haberl, Udo, 52499 Boesweiler (DE)
(74) Representative: Schönbohm, Carla

(57) **Abstract**

The invention relates to peptides capable of binding the TPO receptor of a defined amino acid sequence as well as defined uses thereof.

## Description

Thrombopoietin (TPO), also known as c-Mpl ligand, is a primary regulator of the proliferation and maturation of megakaryocytes as well as of platelet production. Since the direct purification of the protein from plasma, its affinity purification based on its interaction with c-Mpl, and the cloning by various procedures and expression of cDNAs encoding TPO, more than a hundred biological studies of TPO and c-Mpl have been performed with the use of recombinant molecules.

TPO was initially identified as a specific factor for megakaryocytopoiesis and thrombopoiesis. The primary target cell population for TPO in bone marrow comprises megakaryocyte progenitors at the late stage of differentiation, such as colony-forming unit-megakaryocyte (CFU-MK) expressing GpIIb/IIIa (CD41) in the rat, and TPO has been shown to be essential for full maturation of megakaryocytes. In addition to its role in megakaryocytopoiesis, TPO also affects erythroid progenitors in human bone marrow and erythroid and erythromegakaryocytic progenitors in mouse embryonic yolk sac. Furthermore, TPO contributes to production of multiple lineages of hematopoietic cells, as revealed by c-Mpl knockout mice, and it supports primitive hematopoietic progenitors in synergy with other cytokines, as demonstrated with both mouse and human cells. Accordingly, rHuTPO improved the recovery from pancytopenia in myelosuppressed mice. Likewise, a single i.v. administration of PEG-rHuMGDF was able to enhance the recovery from thrombocytopenia as well as both primitive and committed hematopoiesis in myelosuppressed mice (please refer to Kato et al., 1998).

Human TPO cDNA encodes a polypeptide of 353 amino acids. The full-length rHuTPO secreted from mammalian cells after cleavage of the signal peptide consists of 332 amino acids. As with various truncated TPO proteins purified from animals, the entire human TPO molecule is not directly required for biological activity. These observations indicate that the TPO receptor (c-Mpl) binds the N-terminal half of the TPO molecule, which contains an erythropoietin-like domain and retains biological activity. Two disulfide bonds in the N-terminal domain are essential for the biological activity of human and mouse TPO. The C-terminal domain of the protein is highly glycosylated, containing six N-linked and multiple O-linked carbohydrate chains, so that the molecular mass of the full-length rHuTPO derived from CHO cells is between 80 and 100 kDa as determined by SDS-polyacrylamide gel electrophoresis. This glycosylated C-terminal domain is thought to be necessary for survival of TPO in the circulation. In addition, the sugar chains have recently been shown to be important for the secretion of TPO from cells (Kato et al., 1998).

TPO exerts its biological effects through binding to a specific receptor expressed on the cell surface, first identified as the product of the oncogene transduced by the murine myeloproliferative leukaemia (MPL) virus. C-Mpl, the TPO receptor, is a member of the growth hormone subfamily of class I cytokine receptors, which form a homodimeric receptor complex on ligand binding. It has been hypothesized that TPO interacts with at least two identical c-Mpl subunits to generate both proliferation and differentiation signals via the Jak/STAT signalling cascade. TPO probably presents two separate interaction surfaces, one for each receptor subunit (Jagerschmidt et al., 1998).

Since the identification and cloning of TPO, 2 forms of recombinant human TPO, the full-length molecule (rhTPO) and the truncated molecule known as pegylated recombinant human megakaryocyte and development factor (PEG-rhMGDF), have been evaluated in clinical trials for cancer patients with myelosuppressive chemotherapy. Early clinical trials demonstrated their clinical safety and platelet-stimulating activity, leading to attenuate chemotherapy-induced severe thrombocytopenia and reduce the need for platelet transfusions. However, in this setting, the optional schedule of TPO administration depends on the length of the regimen and anticipated timing of platelet nadir. Furthermore, the development of neutralising antibodies and clinically significant thrombocytopenia in some patients who received PEG-rhMGDF has led to discontinuation of its clinical trials. Incidence of such antibodies was 8% and thus much too high to guarantee safe use as a drug (please refer to Cytokines, 2004; page 975).

Although antibodies against rhTPO were non-neutralising and transient, administration of rhTPO might also risk the development of neutralising antibodies against endogenous TPO.

One approach to overcome this antigenic problem of the recombinant TPOs is to generate agonist molecules of a different structure than TPO. One approach aims at the development of agonist antibodies (please refer to Orita et al. 2005) or non-peptide thrombopoietin mimetics (for example WO 00/35446, WO 01/21180, WO 02/085343, WO 04/054515).

Besides non-peptide compounds and antibodies also synthetic peptides were developed to target the TPO receptor. These small-molecular weight substances lack homology with the endogenous physiologic thrombopoietin. These peptide agonists of the thrombopoietin receptor are for example described in WO 96/40750, US 6,121,238, US 6,465,430; WO 98/25965 and WO 00/24770. These TPO mimetic peptides were discovered by screening large phage display libraries of peptides (please refer to Dower et al., 1998 and Cwirla et al., 1997).

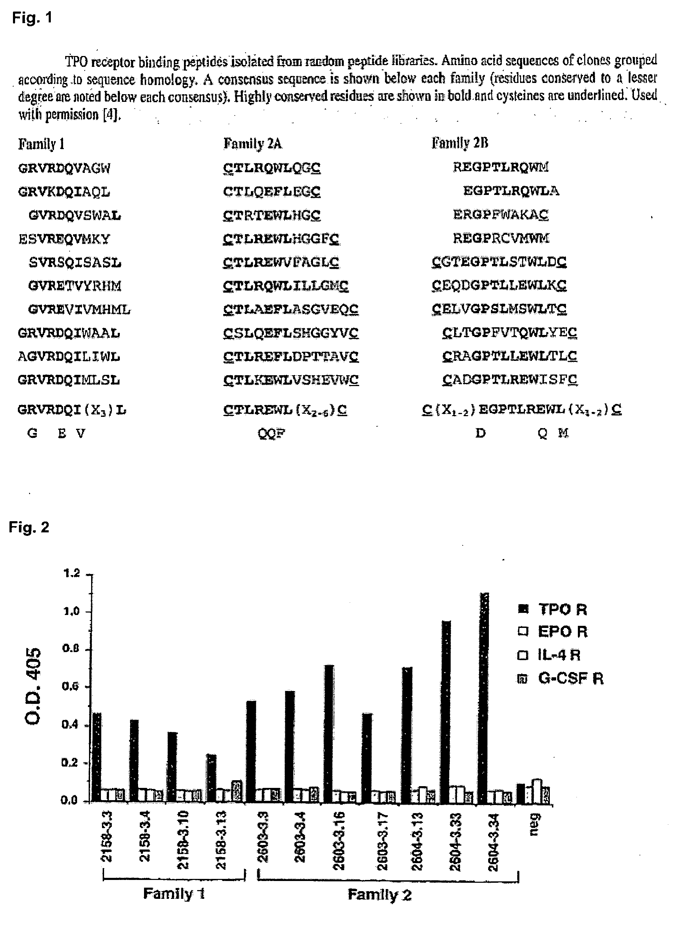

The discovered peptides were categorised into two families based on amino acid sequence similarities. Fig. 1 displays the consensus sequences of the different families as presented in the state of the art (Dower et al., 1998). Family 1 contains the consensus sequence VRDQIXXXL, and family 2 contains the consensus sequence TLREWL. Most of the family 2 sequences were isolated from libraries consisting of random residues flanked by a pair of cysteines, which can form intramolecular disulfide-bonded cyclic structures. Family 2 can be further divided into two subfamilies (A and B as indicated in Fig. 1) with different spacing of the core sequence relative to the first cysteine, and the presence or absence of the dipeptide gly-pro adjacent to a N-terminal side of the core consensus sequence.

Also Kimura et al. (1997) disclose related TPO mimetic peptides, depicting the consensus XXGCXLXXWXXGXC.

As was shown in the state of the art by Dower, 1998, (please refer to Fig. 2), all of the peptides bind to the human (Hu) TPO receptor, but do not bind to the EPO, G-CSF, or interleukin 4 (IL-4) receptors. Thus even though a certain similarity was reported between the TPO mimetic peptides (especially the ones of family 2B) and EPO mimetic peptides (Wrighton et al., 1997) there are apparently structural differences as the known TPO mimetic peptides would not bind the EPO receptor. The TPO mimetic peptides are thus apparently structurally and functional distinctive molecules even though they both depict a gly-pro sequence in their consensus. For EPO mimetic peptides it was surprisingly shown that the proline of the consensus could be replaced by non-conservative amino acid exchanges (please refer to WO 2006/050959).

After the first discovery of the mentioned TPO mimetic peptides, further investigation on these molecules and especially on improved molecules were performed. One PEGylated thrombopoietin peptide mimetic (GW 395058) was further investigated for its therapeutic potential (please refer for example to de Serres et al., 1999; Case et al., 2000).

However, no TPO mimetic peptide is so far clinically approved.

Thus there remains a need to provide additional compounds which have a biological activity of stimulating the production of platelets (thrombopoietic activity) and/or platelet precursor cells, especially megakaryocytes (megakaryopoietic activity). The present invention provides new compounds having such activity (ies), and related aspects.

The present invention provides a novel group of peptides and compounds that are capable of binding to the TPO receptor. In case of TPO agonists they are capable of triggering a transmembrane signal through, i.e., activating, the TPO receptor, which is the same receptor that mediates the activity of endogenous thrombopoietin (TPO). Thus, TPO mimetic compounds according to the present invention depict thrombopoietic activity, i.e., the ability to stimulate, *in vivo* and *in vitro,* the production of platelets, and/or megakaryocytopoietic activity, i.e. the ability to stimulate, *in vivo* and *in vitro,* the production of platelet precursors.

The present invention provides novel peptides which are useful for therapeutic purposes in treating conditions mediated by TPO (for example thrombocytopenia resulting from chemotherapy, radiation therapy, or bone marrow transfusions).

The novel peptides according to the present invention are capable of binding the TPO receptor. It is preferred that they act as TPO receptor agonists which means that they are capable of inducing a signal transduction upon binding the TPO receptor. The peptides according to the present invention are selected from the group consisting of
- peptides comprising the following consensus sequence of amino acids:

   X₅X₆X₇X₈X₉X₁₀X₁₁X₁₂

   wherein each amino acid is selected from natural or unnatural amino acids and
   X₅ is G, A, a conservative exchange of G or Sar,
   X₆ is a charged amino acid,
   X₇ is T, F, K, L, N, Q, R, S or V,
   X₈ is L, C, F, I, M, R, S, V, W or Sec
   X₉ is selected from any amino acid,
   X₁₀ is Q, A, D, E, G, K, M, R, S, T, V, Y or N,
   X₁₁ is W, C, F, G, L, M, S, Y, 1-Nal, 2-Nal, A, L-(benzothienyl)-alanine or 5-MeW, 1-Me-W, or Sec,
   X₁₂ is L, C, G, I, K, M, N, R, V, A, F or Sec and
- functionally equivalent fragments, derivatives and variants of the peptides defined by the above consensus sequence, that bind the TPO receptor and have a charged amino acid in position X₆.

The peptides of the present invention are related to a specific group of TPO mimetic peptides which were categorised in the prior art as subfamily 2B (Dower, 1998). However, in contrast to the peptides described as subfamily 2B, the peptides of the present invention do not carry a proline in position X₆ but a charged amino acid. Surprisingly, it was found with the present invention, that it is possible to introduce a charged amino acid at position X₆. Before the present invention it was only reported to use proline or especially in case of other subtypes of TPO mimetic peptides other amino acids with unpolar side chain such as valine, methionine, leucine or cysteine in this position (Dower 1998, Kimura 1997). However, the introduction of charged amino acids at position X₆ was never reported before.

The inventors have found though that it is possible to introduce in the highly conserved amino acid position X₆ charged amino acids (which constitute a non-conservative exchange of proline) and still obtain peptides that bind the TPO receptor and also very potent TPO mimetic peptides.

In order to enhance the TPO receptor binding and also the TPO mimetic activity, it is preferred that the peptide is dimerised. This means that a peptide is created wherein at least two of the above defined consensus sequences of amino acids are present in the peptide molecule. The monomeric binding units according to the present invention (comprising the above consensus) may also be combined with a monomeric binding unit (consensus sequence) of TPO mimetic peptides known in the state of the art in order to create a functional dimer. Dimerisation of monomeric binding units to create a peptide dimer increases the potency and thus efficacy of the peptides as was reported before in the state of the art (please refer for example to Dower, 1998 and Cwirla, 1997).

Respective dimers are examples of bivalent (dimeric) peptides and exhibit essentially the same biological functions as the monomers but show enhanced biological activity due to a better interaction with the receptor.

Several techniques are known to the person skilled in the art to dimerize or oligomerize peptide monomers which can also be applied according to the teachings of the present invention. Monomers can be dimerized e.g. by covalent attachment to a linker. A linker is a joining molecule creating a covalent bond between the polypeptide units of the present invention. It is e.g. possible to use a biotin/streptavidin system i.e. biotinylating the C-terminus of the peptides and a subsequent incubating the biotinylated peptides with streptavidin. Alternatively, it is known to achieve dimerization by forming a diketopiperazine structure. This method known to the skilled person is described in detail e.g. in Cavelier et al. (in: Peptides: The wave of the Future; Michal Lebl and Richard A. Houghten (eds); American Peptide Society, 2001). The disclosure of these documents regarding the dimerization and a non-covalent multimerization is incorporated herein by reference. Another alternative way to obtain peptide dimers known from prior art is to use bifunctional activated dicarboxylic acid derivatives as reactive precursors of the later linker moieties, which react with N-terminal amino groups, thereby forming the final dimeric peptide. Monomers can also be dimerized by covalent attachment to a linker. Preferably the linker comprises NH-R-NH wherein R is a lower alkylene substituted with a functional group such as carboxyl group or amino group that enables binding to another molecule moiety. The linker might contain a lysine residue or lysine amide. Also PEG may be used a linker. The linker can be a molecule containing two carboxylic acids and optionally substituted at one or more atoms with a functional group such as an amine capable of being bound to one or more PEG molecules.

Suitable methods for dimerising TPO mimetic peptides are also disclosed in Dower et al, 1998 and Cwirla et al, 1997 and WO 00/24770.

A further suitable dimerization method refrains from synthesizing the monomeric peptides forming part of the dimeric peptide in separate reactions prior dimerization or multimerization, but to synthesize the final di- or multimeric peptide in one step as a single continuous peptide; e.g. in one single solid phase reaction. Thus a separate dimerization or multimerization step is obsolete. This aspect provides a big advantage, i.e. the complete and independent control on each sequence position in the final peptide unit. The method allows to easily harbor at least two different receptor-specific binding domains in a peptide unit due to independent control on each sequence position.

According to this embodiment the sequence of the final peptide between the binding domains (which is the "linker region") is composed of amino acids only, thus leading to one single, continuous peptide unit. In a preferred embodiment of the invention the linker is composed of natural or unnatural amino acids which allow a high conformational flexibility. In this regard it can be advantageous to use glycine residues as linking amino acids, which are known for their high flexibility in terms of torsions. However, also other amino acids, such as alanine or beta-alanine, or a mixture thereof can be used. The number and choice of used amino acids depend on the respective steric facts. This embodiment allows the custom-made design of a suitable linker by molecular modeling in order to avoid distortions of the bioactive conformation and thus allows perfect matching with the receptor units. A linker composed of 3 to 5 amino acids is especially preferred.

It is noteworthy that the linker between the functional binding domains (also referred to as monomeric units) of the peptide units can be either a distinct part of the peptide or can be composed - fully or in parts - of amino acids which are part of the monomeric binding domains. Thus the term "linker" is rather defined functionally than structurally, since an amino acid might form part of the linker unit as well as of the monomeric binding subunits.

The dimeric peptides according to the invention show much higher activity then the corresponding monomeric peptides and thus confirm the observation known from other dimeric peptides (e.g. TPO mimetic peptides as described in Cwirla et al, 1997) that an increase of efficacy is associated with bivalent peptide concepts.

Examples for dimerization methods being applied to the monomers of the peptide units include (but are not limited to):
1. The dimerization via connection from C-terminus to C-terminus wherein the C-terminus of one of said monomeric peptide is covalently bound to the C-terminus of the other peptide. The linker/spacer between the monomers can contain a diketopiperazine unit. A preferred Gly-Gly diketopiperazine scaffold can be achieved by activating the C-terminal glycine monomer. This principle can also be use for forming a C-terminal dimerization. 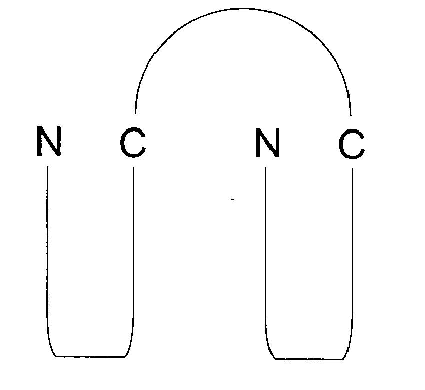
2. The dimerization via connection from N-terminus to N-terminus wherein the N-terminus of one of said monomeric peptides is covalently bound to the N-terminus of the other peptide, whereby the spacer unit is preferably containing a dicarboxylic acid building block. 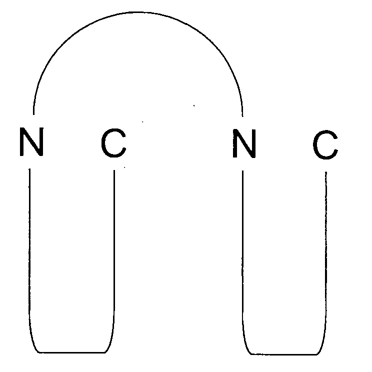
3. The dimerization via the side chains wherein an amino acid side chain of one of said monomeric peptides is covalently bound to an amino acid side chain of the other peptide with inclusion of a suitable spacer molecule connecting the two peptide monomers. 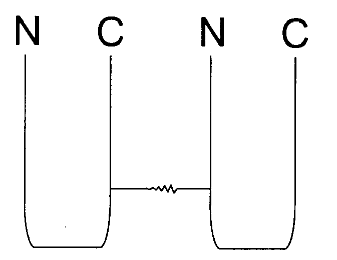 This can include:
   (a) the connection via an amide bond.
   (b) or the connection via a disulfide bridge.
   According to this strategy, the covalent bridge linking the peptide monomers to each other thereby forming the peptide unit is formed between the sidechains of the C-terminal amino acid of the first monomeric peptide unit and the N-terminal amino acid of the second peptide monomer. Hence, it is preferred according to this dimerization strategy that the monomeric peptides to be dimerized carry an amino acid with a bridge forming functionality at either the N- or C-terminus thereby allowing the formation of a covalent bond between the last amino acid of the first peptide and the first amino acid of the second peptide. The bond creating the dimer is preferably covalent. Suitable examples of respective bridges are e.g. the disulfide bridge and the diselenide bridge. However, also e.g. amide bonds between positively and negatively charged amino acids or other covalent linking bonds such as thioether bonds are suitable as linking moieties.
   Preferred amino acids suitable for forming respective connecting bridges between the monomeric binding units to form the final peptide unit are e.g. cysteine, cysteine derivatives such as homocysteine or selenocysteine or thiolysine. They form either disulfide bridges or, in case of selenium containing amino acids, diselenide bridges.
   According to a further development either at the N-or the C-terminus of the peptide dimer (and hence of the respective monomeric peptide units either being located at the beginning or the end of the dimer) comprise an extra amino acid, allowing the coupling of the polymeric carrier such as HES in order to create the supravalent compound. Consequently, the introduced amino acid carries a respective coupling functionality such as e.g. an SH-group. One common example for such an amino acid is cysteine. However, also other amino acids with a functional group allowing the formation of a covalent bond (e.g. all negatively and positively charged amino acids) are suitable.
   According to a further development the amino acid at the C and/or the N terminus involved in forming the covalent bridge for connecting the monomeric units to a dimer depicts a charged group such as e.g. the COO- or the NH₃⁺ group. This feature leads to a favourable stabilisation of the structure of the intermolecular bridge
4. The dimerization by forming continuous bi- or multivalent peptides was already outlined above.

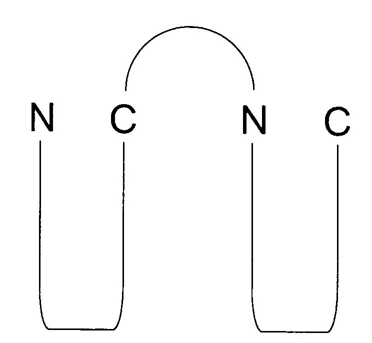

The monomers and di- or multimeric peptides can be modified by e.g. acetylation or amidation or be elongated at C-terminal or N-terminal positions. Extension with one or more amino acids at one of the two termini (N or C), e.g. for preparation of an attachment site for the polymeric carrier often leads to a heterodimeric bivalent peptide unit which can best be manufactured as a continuous peptide. The reactive side chains of the peptides may also serve as a linking tie e.g. for further modifications. The peptide units furthermore optionally comprise intramolecular bridges between amino acids having a bridge forming side chain functionality such as e.g. the cysteines.

Also provided with the present invention is a method for dimerising monomeric peptide units comprising an amino acid consensus sequence as defined in the claims and above to form a TPO mimetic dimer, wherein said dimer is created by forming a covalent bond between the monomeric peptide units, wherein said bond is formed between the C-terminal amino acid of the first monomeric peptide unit and the N-terminal amino acid of the second monomeric peptide unit.

According to a further embodiment monomeric peptide units are used, carrying amino acid at either the C- or the N-terminus having a side chain functionality capable of forming a covalent bond, wherein a covalent bond is formed between the side chain of the C-terminal amino acid of the first monomeric peptide unit and the side chain of the N-terminal amino acid of the second monomeric peptide units.

Due to the fact that the potency is increased by dimerisation it is preferred that the peptides are present in at least dimeric form.

Enlarged versions of the consensus amino acid sequence, where suitable amino acids are defined for positions surrounding the above consensus sequence are defined in the dependent claims and are also described below. Please note that the numbering of amino acids used in the present application (X₅, X₆, X₇ etc.) was only provided in order to alleviate the comparison between the peptides of the present invention and the TPO mimetic peptides known in the state of the art. However, this numbering does not refer to the overall length of the peptides and hence shall also not imply that it is always necessary that all positions are occupied besides the defined consensus sequence of claim 1 or that they constitute the overall length. It is for example not necessary that position X₁ is occupied or position X₁₇. For example, a peptide starting with X₅ may also bind the TPO receptor and may also act as a TPO agonist especially if formulated as a dimer. Consequently, the numbering of the amino acid positions used in this application shall apply no restrictions regarding the size of the peptides that shall only alleviate comparison of the peptides with the prior art.

Having this in mind the peptides according to the present invention may comprise additional amino acid positions surrounding the above defined core consensus sequence.

According to one embodiment at least one of the following amino acid positions may be additionally present in the above defined consensus sequence:

X₁, X₂, X₃, X₄

As the consensus sequence as defined in claim 1 is pre-dominantly decisive for the TPO receptor binding properties and in case of TPO agonists the TPO mimetic activity, the amino acids for the above positions X₁ to X₄ may be selected from any natural or unnatural amino acid (including D amino acids). However, according to one embodiment they are chosen as follows:
In case X₁ is present, X₁ may be L, G, or an amino acid depicting a side chain functionality capable of forming a covalent bond such as for example cysteine, homocysteine or selenocysteine.
In case X₂ is present it may be selected from the group consisting of H, A, N, G, T, I or an amino acid depicting a reactive side chain functionality capable of forming a covalent bond such as for example cysteine, homocysteine or selenocysteine.
In case X₃ is present it may be selected from the group consisting of A, C, E, G, I, L, M, P, R, Q, S, T or V.
In case X₄ is present it may selected from the group consisting of A, C, D, E, K, L, Q, R, S, T, V or G.

The peptide consensus may also be enlarged in the C-terminal direction of the amino acid consensus. The consensus may thus comprise additionally at least one of the following amino acid positions:

X₁₃X₁₄X₁₅X₁₆X₁₇X₁₈X₁₉

Again, X₁₃ to X₁₉ may be selected from any natural or unnatural amino acid (including D amino acids). However, they are preferably defined as follows:
lf X₁₃ is present, X₁₃ is L, H, S, A, Ava, Abu, I, V, L, F, T, K or E,
If X₁₄ is present, X₁₄ is G, F, A, diphenylalanine, I, V, L, S, Q, C or an amino acid depicting a side chain functionality capable of forming a covalent bond,
If X₁₅ is present, X₁₅ is G, C, selenocysteine, N, R, p-amino phenylalanine, Ac-K, R, Ava, K, T, V, Q or an amino acid depicting a side chain functionality capable of forming a covalent bond,
If X₁₆ is present, X₁₆ may be F, G, A, beta-A, N-methyl-A, Sar, I, V, L, T, R or E,
lf X₁₇ is present, X₁₇ may be C, G, beta-A, K, D or R,
If X₁₈ is present, X₁₈ may be G, K, beta-A, D, N, R or A,
If X₁₉ is present, X₁₉ may be G, P, T, N or R.

Peptides and compounds of the present invention may be linear or cyclic. According to a further embodiment of the present invention the peptide is cyclised. By "cyclic" is meant that at least two separated, i.e., non-continuous, portions of the molecule are linked to each other. For example, the amino and carboxy terminus of the ends of the molecule could be covalently linked to form a cyclic molecule. Alternatively, the molecule could contain two or more amino acids capable or forming a covalent bond (for example cysteine or homocysteine residues), which could cyclise via disulfide due to suitable side chain functionalities bond formation or via formation of a different covalent bond (e.g. amide or selenide bond).

Thus the peptides of the present invention may be present in a cyclised form with an intramolecular covalent bond such as a disulfide bond between the thiol groups of the cysteines or likewise amino acids carrying a thiol group. Also, an intermolecular disulfide bond between the thiol groups of the cysteines can be produced to yield a dimeric (or higher oligomeric) compound. One or more of the cysteine residues may also be substituted with a different suitable amino acid such as homocysteine or thiolysine.

Other embodiments of this invention provide for analogues of these disulfide derivatives in which one of the sulfurs has been replaced by a CH₂-group or another isoster for a sulfur.

Cyclisation is preferably achieved between two amino acids of the monomeric TPO binding units depicting a side chain functionality capable of forming a covalent bond. Said amino acids are capable of forming an intramolecular bridge by forming a covalent bond between their side chains.

Examples for suitable bridging units are the disulfide bridge and the diselenide bridge. Suitable examples of amino acids depicting such bridge forming functionalities in their side chain are for example cysteine und cysteine derivatives such as homocysteine or selenocysteine but also thiolysine. The formation of a diselenide bridge for example between selenocysteine residues might even have advantages over a cysteine bridge. This as a selenide bridge is more stable and reducing environments. The conformation of the peptide might thus even be preserved under more difficult conditions.

However, it is evident, that also amino acids are suitable for forming an intramolecular bridge which depict a side chain with the functionality allowing the formation of different covalent bonds such as for example an amide bond between an amino acid having a positively charge side chain (for example K, H, R or ornithine, Dap or Dab) and an amino acid having a negatively charge side chain (for example the proteinogenic amino acids D or E). Further examples are amide and thioether bridges.

In order to facilitate the formation of intramolecular sulfide bridges several methods are known in the state of the art which allow cyclisation of peptides for example by formation of intramolecular disulfide bridges. According to a standard method K₃[Fe(CN)₆] is used as an oxidation agent. This reagent provides clean cyclisation products with a high yield; side reactions are avoided. Furthermore, cyclisation methods are known which employ the use of oxygen or iodine. A further method for cyclisation of peptide uses immobilised Ellmann's-reagent (5, 5;-dithiobis(2-nitrobenzoic acid)) as oxidation reagent. A further method for formation of disulfide bridges for cyclisation of peptides employs the use of DMSO as an oxidation agent.

Some of the known suitable methods are also outlined in the example/production section.

According to one embodiment said covalent bond for forming an intramolecular bridge is created between two amino acids of the peptide sequence that are separated by twelve amino acids. It is assumed that this spacing leads to a beneficial three dimensional conformation for binding the TPO receptor.

Accordingly, according to one embodiment the peptide has the following structure:

CA(AS₁₋₂)X₄-X₁₂(AS₁₋₂)CA

wherein "CA" represents an amino acid involved in forming a covalent bond for cyclisation of the peptide and AS₁₋₂ means that there may be one or two amino acids present (especially X₂ and/or X₃ and X₁₃ and/or X₁₄).

The covalent bond is formed between the amino acids defined as "CA". In case only one amino acid is present in the defined N-terminal portion of the peptide consensus, it is preferred that two amino acids are present in the C-terminal portion of the defined peptide consensus adjacent to the amino acid "CA" as defined above and vice versa. This has the effect that twelve amino acids are positioned between the two amino acids "CA" forming the covalent intramolecular bond.

Further embodiments of the present invention are outlined below:
In case X₁ is present in the consensus sequence it is preferably an amino acid depicting a side chain functionality capable of forming a covalent bond such as cysteine, homocysteine or selenocysteine.
In case X₂ is present in the peptide consensus it is preferably an amino acid depicting a side chain functionality capable of forming a covalent bond in case X₁ is not an amino acid depicting a side chain functionality capable of forming a covalent bond. A suitable example is again cysteine and related amino acids.
In case X₃ is present in the molecule it is preferred that it is alanine.
In case X₄ is present in a molecule it may be D, E or K.

It is preferred that X₅ is glycine.

X₆ is either a negatively or a positively charged amino acid. A positively charged amino acid such as basic amino acids as for example the proteinogenic amino acids K, R and H can be used in this position. However, the charged amino acid can also be a non-proteinogenic natural or non-natural amino acid. Also comprised are respective analogues of the mentioned amino acids. Also non-proteinogenic positively charged amino acids may be used which have a side chain which is elongated compared to lysine. One example is homoarginine. Also negatively charged amino acids such as D or E may be used as a charged amino acid in position X₆. Furthermore, also non-proteinogenic and also non-natural amino acids (including D amino acids) may be used in this position X₆. One further example is alpha-amino adipic acid (Aad).

In case X₇ is present it is preferably F, R, S or T.

In case X₈ is present it is preferably F, L, V or W.

In case X₉ is present is preferably A, K, L, M, R, S, V, T, E, G, H, P, Q, W, Nle, Ac-K and more preferably A, K, L, M, R, S, V or T and most preferred R.

X₁₀ may be E, G, K, M, Q, R, S or T and more preferably E or Q.

X₁₁ is also an important amino acid position of the peptide consensus. Generally, L as well as D amino acids may be present in position X₁₁ (e.g. D-W or D-1-Nal). It is preferably occupied by W, F, L, A, 1-Nal or 2-Nal, L-(benzothienyl)-alanine or respective derivatives such as 5-Me-W or 1-Me-W. The amino acids may be present in D form.

X₁₂ is preferably C, I, K, L, M or V and more preferably I, K, L, M or V or I or L.

X₁₄ is preferably an amino acid having a side chain functionality capable of forming a covalent bond in case X₁₅ is not an amino acid having a side chain functionality capable of forming a covalent bond (e.g. cysteine, homocysteine).

Correspondingly, X₁₅ is preferably an amino acid having a side chain functionality capable of forming a covalent bond in case X₁₄ is not an amino acid having a side chain functionality capable of forming a covalent bond.

The length of the overall peptide is preferably between 8 or 10 to 50 or 60 amino acids. In preferred embodiments, the peptide consensus depicts a length of at least 8 amino acids, 8 or 10 to 50, 8 or 10 to 40, 8 or 10 to 45, 8 or 10 to 30, 8 or 10 to 25, 8 or 10 to 20, 8 or 10 to 15 or 8 or 10 to 14 amino acids. Of course the consensus can be embedded respectively be comprised by longer amino acid sequences. A longer length of the overall peptide can also be created by dimerising two monomeric TPO receptor binding peptide units of the above defined consensus sequences as already described above.

As dimeric peptides are preferred due to their enhanced activity, according to one embodiment the invention provides a TPO mimetic peptide, comprising at least two monomeric TPO mimetic peptide consensus sequences, wherein at least one of the monomeric consensus sequences comprises an amino acid consensus sequence as described above.

As outlined above, oligomerization of the monomeric peptides to dimers or even multimers usually improves the TPO receptor binding properties and hence also the TPO mimetic agonist activity compared to the respective monomeric peptides as was also observed with EPO mimetic peptides. However, it is desirable to further enhance the TPO mimetic activity.

Several approaches were made in the prior art in order to increase the activity of the peptides, for example by variation of the amino acid sequence in order to identify more potent candidates. However, so far it is still desirable to further enhance the activity of TPO mimetic peptides in order to improve the biological activity.

A further embodiment of the present invention provides a solution to that problem. Therein a TPO mimetic compound is provided that binds target molecules and comprises
i) at least two peptide units wherein each peptide unit comprises at least two domains with a binding capacity to the target;
ii) at least one polymeric carrier unit;
wherein said peptide units are attached to said polymeric carrier unit and wherein at least one domain of at least one peptide unit comprises an amino acid consensus sequence according to the present invention.

Surprisingly, it has been found that the combination of two or more preferably dimeric peptides according to the invention on a polymeric support is greatly increasing the efficacy of the di- (or even multimeric) peptides to the TPO receptor not only additively, but even over-additively. Thus a synergistic effect is observed.

The term "bivalent" as used herein is defined as a peptide comprising two domains with a binding capacity to a target, here the TPO receptor. It is used interchangeably with the term "dimeric". Accordingly, a "multivalent" or "multimeric" TPO mimetic peptide has several respective binding domains for the TPO receptor. It is self-evident that the terms "peptide" and "peptide unit" do not incorporate any restrictions regarding size and incorporate oligo- and polypeptides as well as proteins. However, it is preferred that the peptide units coupled to the carrier unit have a length of about 200 amino acids or less, or of about 150 amino acids or less, more preferred about 100 amino acids or even 50 amino acids and less.

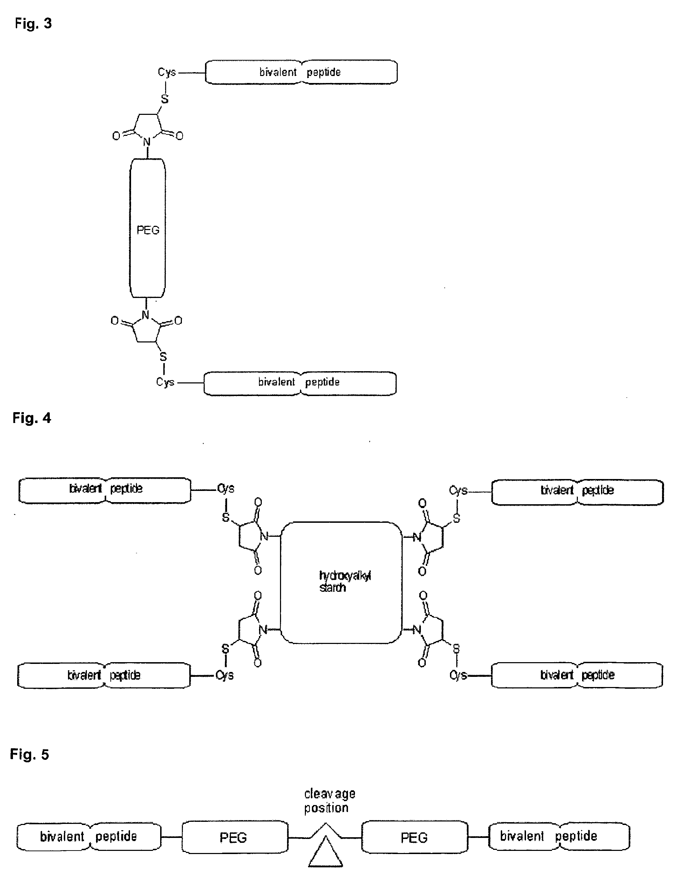

Compounds comprising two or more bi- or multivalent peptide units attached to a polymeric carrier unit are named "supravalent" in the context of the present invention. These supravalent molecules greatly differ from the dimeric TPO mimetic molecules known in the state of the art. The state of the art combines several merely monomeric peptide units in order to create a dimer. In contrast, the supravalent molecules are generated by connecting already (at least) bivalent peptide units to a polymeric carrier unit thereby creating a supravalent molecule carrying several di- or multimeric peptide units (illustrated examples of this concept are given in figs. 3 and 4). Thereby the overall activity and efficacy of the di- or multimeric peptides is greatly enhanced thus decreasing the EC50 dose.

So far the reasons for the great potency of the supravalent molecules compared to the bi- or multimeric molecules known in the state of the art are not fully understood. It might be due to the fact that the dimeric molecules known in the state of the art (e.g. dimeric TPO mimetic peptides) provide merely one target respectively one active receptor binding unit per compound molecule. Thus only one (dimeric) receptor complex is generated upon binding of the dimeric compound thereby inducing only one signal transduction process in the cell. For example two monomeric TPO mimetic peptides are connected via a linker to form a peptide dimer thereby facilitating dimerization of the receptor monomers necessary for signal transduction (Dower et al, 1998).

In contrast, the supravalent compounds according to the invention comprise several already di- or multimeric receptor binding units. Supravalent compounds according to the present invention thus carry several (bi- or multivalent) peptides binding the TPO receptor and thus receptor binding units. Each di- or multimeric peptide unit coupled to the carrier represents one receptor binding unit. This might allow the generation of several receptor complexes on the cell surface per compound molecule thereby inducing (or blocking in case of an antagonist) several signal transductions and thereby potencing the activity of the peptide units over-additively. Binding of the supravalent compounds might result in a clustering of receptor complexes on the cell-surface.

The polymeric carrier unit comprises at least one natural or synthetic branched, linear or dendritic polymer. The polymeric carrier unit is preferably soluble in water and body fluids and is preferably a pharmaceutically acceptable polymer. Water soluble polymer moieties include, but are not limited to, e.g. polyalkylene glycol and derivatives thereof, including PEG, PEG homopolymers, mPEG, polypropyleneglycol homopolymers, copolymers of ethylene glycol with propylene glycol, wherein said homopolymers and copoloymers are unsubstituted or substituted at one end e.g. with an acylgroup; polyglycerines or polysialic acid; carbohydrates, polysaccharides, cellulose and cellulose derivatives, including methylcellulose and carboxymethylcellulose; starches (e.g. hydroxyalkyl starch (HAS), especially hydroxyethyl starch (HES) and dextrines, and derivatives thereof; dextran and dextran derivatives, including dextransulfat, crosslinked dextrin, and carboxymethyl dextrin; chitosan (a linear polysaccharide), heparin and fragments of heparin; polyvinyl alcohol and polyvinyl ethyl ethers; polyvinylpyrrollidon; alpha,beta-poly[(2-hydroxyethyl)-DL-aspartamide; and polyoxyethylated polyols. One example of a carrier unit is a homobifunctional polymer, of for example polyethylene glycol (bis-maleimide, bis-carboxy, bis-amino etc.).

The polymeric carrier unit which is coupled to the peptide units according to the present invention can have a wide range of molecular weight due to the different nature of the different polymers that are suitable in conjunction with the present invention. There are thus no size restrictions. However, it is preferred that the molecular weight is at least 3 kD, preferably at least 10kD and approximately around 20 to 500 kD and more preferably around 30 to 150 or around 60 or 80 kD. The size of the carrier unit depends on the chosen polymer and can thus vary. For example, especially when starches such as hydroxyethylstarch are used, the molecular weight might be considerably higher. The average molecular weight might then be arranged around 100 to 4,000 kD or even be higher. However, it is preferred that the molecular weight of the HES molecule is about 100 to 300 or 500kD, preferably around 150 - 250 or 200kD. The size of the carrier unit is preferably chosen such that each peptide unit is respectively can be optimally arranged for binding the TPO receptor.

In order to facilitate this, one embodiment of the present invention uses a carrier unit comprising a branching unit. According to this embodiment, the polymers, as for example PEG, are attached to a branching unit thus resulting in a large carrier molecule allowing the incorporation of numerous peptide units. Examples for appropriate branching units are glycerol or polyglycerol. Also dendritic branching units can be used as for example taught by Haag 2000, herein incorporated by reference. Also the HES carrier may be used in a branched form. This e.g. if it is obtained to a high proportion from amylopectin.

Preferably after the peptide units are created by combining the monomeric binding units (either head to head, head to tail, or tail to tail) to peptide units the polymeric carrier unit is connected/coupled to the peptide units. The polymeric carrier unit is connected to the peptide units via a covalent or a non-covalent (e.g. a coordinative) bond. However, the use of a covalent bond is preferred.

The attachment can occur e.g. via a reactive amino acid of the peptide units e.g. lysine, cysteine, histidine, arginine, aspartic acid, glutamic acid, serine, threonine, tyrosine or the N-terminal amino group and the C-terminal carboxylic acid. In case the peptide does not carry a respective reactive amino acid, such an amino acid can be introduced into the amino acid sequence. The coupling should be chosen such that the binding to the target is not or at least as little as possible hindered. Depending on the conformation of the peptide unit the reactive amino acid is either at the beginning, the end or within the peptide sequence.

In case the polymeric carrier unit does not possess an appropriate coupling group, several coupling substances/linker can be used in order to appropriately modify the polymer in order that it can react with at least one reactive group on the peptide unit to form the supravalent compound. Suitable chemical groups that can be used to modify the polymer are e.g. as follows:
• Acylating groups which react with the amino groups of the protein, for example acid anhydride groups, N-acylimidazole groups, azide groups, N-carboxy anhydride groups, diketene groups, dialkyl pyrocarbonate groups, imidoester groups, and carbodiimide-activated carboxyl-groups. All of the above groups are known to react with amino groups on proteins/peptides to form covalent bonds, involving acyl or similar linkages;
• alkylating groups which react with sulfhydryl (mercapto), thiomethyl, imidazo or amino groups on the peptide unit, such as halo-carboxyl groups, maleimide groups, activated vinyl groups, ethylenimine groups, aryl halide groups, 2-hydroxy 5-nitro-benzyl bromide groups; and aliphatic aldehyde and ketone groups together with reducing agents, reacting with the amino group of the peptide;
• ester and amide forming groups which react with a carboxyl group of the peptide, such as diazocarboxylate groups, and carbodiimide and amine groups together;
• disulfide forming groups which react with the sulfhydryl groups on the protein, such as 5,5'-dithiobis (2-nitrobenzoate) groups, ortho-pyridyl disulfides and alkylmercaptan groups (which react with the sulfhydryl groups of the peptide in the presence of oxidizing agents such as iodine);
• dicarbonyl groups, such as cyclohexandione groups, and other 1,2-diketone groups which react with the guanidine moieties of protein;
• diazo groups, which react with phenolic groups on the peptide;
• reactive groups from reaction of cyanogens bromide with the polysaccharide, which react with amino groups on the protein.

Thus in summary, the compound according to the invention may be made by - optionally - first modifying the polymeric carrier chemically to produce a polymeric carrier having at least one chemical group thereon which is capable of reacting with an available or introduced chemical group on the peptide unit, and then reacting together the - optionally - modified polymer and the peptide unit to form a covalently bonded complex thereof utilising the chemical group of the - if necessary - modified polymer.

In case coupling occurs via a free SH-group of the peptide, the use of a maleimide group in the polymer is preferred.

In order to generate a defined molecule it is preferred to use a targeted approach for attaching the peptide units to the polymeric carrier unit. In case no appropriate amino acids are present at the desired attachment site, appropriate amino acids should be incorporated in the peptide unit. For site specific polymer attachment a unique reactive group e.g. a specific amino acid at the end of the peptide unit is preferred in order to avoid uncontrolled coupling reactions throughout the peptide leading to a heterogeneous mixture comprising a population of several different polymeric molecules.

The coupling of the peptide units to the polymeric carrier unit, e.g. PEG or HES, is performed using reactions principally known to the person skilled in the art. E.g. there are number of PEG and HES attachment methods available to those skilled in the art (see for example WO 2004/100997 giving further references, Roberts et al., 2002; US 4,064,118; EP 1 398 322; EP 1 398 327; EP 1 398 328; WO 2004/024761; WO 98/25965 and especially WO2006/050959 all herein incorporated by reference).

It is important to understand that the concept of supravalency described herein is different from the known concepts of PEGylation or HESylation which are, however, also within the scope of the present invention. In the state of the art e.g. PEGylation is only used in order to produce either peptide dimers or in order to improve pharmacokinetic parameters by attaching one or more PEG units to a peptide. However, as outlined above, the attachment of two or more at least bivalent peptide units to e.g. HES as a polymeric carrier unit also greatly enhances efficacy (thus decreasing the EC50-dose). The concept of this invention thus has strong effects on pharmacodynamic parameters and not only on pharmacokinetic parameters as it is the case with the PEGylation or HESylation concepts known in the state of the art. However, of course the incorporation of for example PEG or HES as polymeric carrier unit also has the known advantages regarding pharmacokinetics.

PEGylation is usually undertaken to improve the biopharmaceutical properties of the peptides. The most relevant alterations of the protein molecule following PEG conjugation are size enlargement, protein surface and glycosylation function masking, charge modification and epitope shielding. In particular, size enlargement slows down kidney ultrafiltration and promotes the accumulation into permeable tissues by the passive enhance permeation and retention mechanism. Protein shielding reduces proteolysis and immune system recognition, which are important routes of elimination. The specific effect of PEGylation on protein physicochemical and biological properties is strictly determined by protein and polymer properties as well as by the adopted PEGylation strategy.

However, the use of PEG or other non-biodegradable polymers might lead to new problems.

During in vivo applications, dosage intervals in a clinical setting are triggered by loss of effect of the drug. Routine dosages and dosage intervals are adapted such that the effect is not lost during dosage intervals. Due to the fact that peptides attached to a non-biodegradable, large polymer unit (e.g. a PEG-moiety) can be degraded faster than the support molecule can be eliminated by the body, a risk of accumulation of the carrier unit can arise. Such a risk of accumulation always occurs as effect-half life time of the drug is shorter than elimination half life time of the drug itself or one of its components/metabolites. Thus, accumulation of the carrier molecule should be avoided especially in long-term treatments because peptides are usually PEGylated with very large PEG-moieties (~20-40kD) which thus show a slow renal elimination. The peptide moiety itself undergoes enzymatic degradation and even partial cleavage might suffice to deactivate the peptide.

In order to find a solution to this potential problem one embodiment of the present invention teaches the use of a polymeric carrier unit that is composed of at least two subunits. The polymeric subunits are connected via biodegradable covalent linker structures. According to this embodiment the molecular weight of the large carrier molecule (for example 40 kD) is created by several small or intermediate sized subunits (for example each subunit having a molecular weight of 5 to 10 kD), that are connected via biodegradable linkers. The molecular weights of the modular subunits add up thereby generating the desired molecular weight of the carrier molecule. However, the biodegradable linker structures can be broken up in the body thereby releasing the smaller carrier subunits (e.g. 5 to 10 kD). The small carrier subunits show a better renal clearance than a polymer molecule having the overall molecular weight (e.g. 40kD). An illustrating example is given in Fig. 5.

The linker structures are selected according to known degradation properties and time scales of degradation in body fluids. The breakable structures can, for instance, contain cleavable groups like carboxylic acid derivatives as amide/peptide bonds or esters which can be cleaved by hydrolysis (see e.g. Roberts, 2002 herein incorporated by reference). PEG succinimidyl esters can also be synthesized with various ester linkages in the PEG backbone to control the degradation rate at physiological pH (Zhao, 1997, herein incorporated by reference). Other breakable structures like disulfides of benzyl urethanes can be cleaved under mild reducing environments, such as in endosomal compartments of a cell (Zalipsky, 1999) and are thus also suitable. Other criteria for selection of appropriate linkers are the selection for fast (frequently enzymatic) degradation or slow (frequently non-enzymatic decomposition) degradation. Combination of these two mechanisms in body fluids is also feasible. It is clear that this highly advantageous concept is not limited to the specific peptide units described or referred to herein but also applies to other pharmaceutical molecules that are attached to large polymer units such as PEG molecules wherein the same problems of accumulation arises.

According to one embodiment hydroxyalkylstarch and preferably HES is used as polymeric carrier unit. HES has several important advantages. First of all, HES is biodegradable. Furthermore, the biodegradability of HES can be controlled via the ratio of hydroxyethyl groups and can thus be influenced. A molar degree of substitution of 0.4-0.8 (in average 40-80% of the glucose units contain a hydroxyethyl group) are well suitable for the purpose of the present invention. Due to the biodegradability, accumulation problems as described above in conjunction with PEG do usually not occur. Furthermore, HES has been used for a long time in medical treatment e.g. in form of a plasma expander. Its innocuousness is thus approved.

Furthermore, derivatives of hydrolysis products of HES are detectable by gas chromatography. HES-peptide conjugates can be hydrolysed under conditions under which the peptide units are still stable. This allows the quantification and monitoring of the degradation products and allows evaluations and standardisations of the active peptides.

According to a further embodiment a first type of polymeric carrier unit is used and loaded with peptide units. This first carrier is preferably easily biodegradable as is e.g. HES. However, not all attachment spots of the first carrier are occupied with peptide units but only e.g. around 20 to 50%. Depending on the size of the used polymer, several hundred peptide units can be coupled to the carrier molecule. However, depending on the peptide used usually less peptide units (2 to 50, 2 to 20, 2 to 10 or 3 to 5) are coupled. The rest (or at least some) of the remaining attachment spots are occupied with a different carrier, e.g. small PEG units having a lower molecular weight than the first carrier. This embodiment has the advantage that a supravalent composition is created due to the first carrier which is however, very durable due to the presence of the second carrier, which is constituted preferably by PEG units of 3 to 5 or to 10kD. However, the whole entity is very well degradable, since the first carrier (e.g. HES) and the peptide units are biodegradable and the second carrier, e.g. PEG is small enough to be easily cleared from the body.

The monomers constituting the binding domains of the peptide units recognize the homodimeric thrombopoietin receptor. The latter property of being a homodimeric receptor differentiates the TPO-receptor from many other cytokine receptors. The peptide units comprising at least two TPO mimetic monomeric binding domains as described above bind the TPO receptor and preferably are able to di- respectively multimerise their target and/or stabilize it accordingly, thereby creating a signal transduction inducing complex.

As outlined above, the concept of supravalency has several advantages. However, it is also within the scope of the present invention to couple only one carrier unit to the peptides of the present invention, for example in order to increase the molecular weight of the peptides or for example in order to protect the peptides from degradation (see above). Also within the scope of the present invention is the attachment of two or more carrier units to the peptides of the present invention.

Suitable carriers were already outlined above in the context of the supravalence concept and thus comprise for example polymeric carriers such as polyalkylene glycol and derivatives thereof, polyglycerines or polysialic acid; carbohydrates and polysaccharides, especially starches such as hydroxyalkyl and especially hydroxyethyl starches; dextran and the like (see above).

According to a further embodiment, the peptides of the present invention are fused to at least one Fc group either directly or through one or more linker groups. The Fc group serves as a kind of carrier.

The Fc sequence may be selected from the human immunoglobulin IgG-1 heavy chain or any other Fc sequence known in the art (for details please refer for example to WO 00/24770).

It is well known that Fc regions of antibodies are made up of monomeric polypeptides segments that may be linked into dimeric or multimeric forms by disulfide bonds or by non-covalent association. The number of intermolecular disulfide bonds between monomeric subunits of native Fc molecules ranges from 1 to 4 depending on the class (for example IgG, IgA, IgE) or their respective subclasses. The term "Fc" as used herein is generic to the monomeric, dimeric and multimeric forms of Fc molecules. It should be noted that Fc monomers will usually spontaneously dimerise either by formation of covalent bonds between appropriate cysteine residues or by non-covalent interactions. The term "Fc" herein is used to mean any of these forms: the native monomer, the native dimer (disulfide bond linked), modified dimers (disulfide and/or non-covalently linked), and modified monomers such as derivatives. Also variants, analogues or derivatives of the Fc portion are comprised by the scope of the present invention. Details of respective variants, analogues or derivatives are described in detail in WO 00/24770, herein fully incorporated by reference).

The Fc fusions may be at the N- or C-terminus of the TPO receptor binding peptides according to the present invention. However, they may also be incorporated at both termini of the TPO receptor binding peptides (either to their monomeric or dimeric form). The incorporation of the Fc part, especially at the N-terminus of the TPO mimetic peptide, is preferred as the bioactivity is increased.

Respective fusions may be for example produced recombinantly.

The present invention also comprises respective compound production methods, wherein the peptide units are connected to the respective carrier units. The present invention furthermore comprises respective compound production methods, wherein the peptide units are connected to the respective polymeric carrier unit.

Please note, that fragments, derivatives and variant polypeptides of the described peptides according to the present invention retain substantially the same biological function or activity as the TPO receptor binding or TPO mimetic peptides described herein. As described above, the peptides according to the present invention carry the charged amino acid in position X₆, a feature which was not known in the state of the art.

A fragment is less than a full length peptide (or polypeptide, the term peptide as used herein does not comprise any size restrictions), which retains substantially the similar or same functional activity.

Derivatives include peptides that have been chemically modified to provide an additional structure and/or function.

Derivatives can be modified by either natural processes or by chemical modification techniques, both of which are well known in the art. Modifications can occur anywhere in a polypeptide, including the peptide backbone, the amino acid side-chains, and the amino or carboxyl termini.

Other chemical modifications include e.g. acetylations, acylation, amidation, covalent attachment of different chemical moieties, cross-linking, cyclization, disulfide bond or other bridge formations, hydroxylation, methylation, oxidation, PEGylation, selenoylation.

Variants of peptides according to the present invention include polypeptides having one or more amino acid sequence exchanges with respect to the amino acids defined in the consensus. Of course, the may also contain amino acids other than natural amino acids such as unnatural and D-amino acids.

E.g., one or more conservative amino acid substitutions can be carried out within the amino acid sequence of the polypeptides according to this invention in order to arrive at functional variants of the different embodiments of the invention as described above. The substitution occurs e.g. within amino acids having unpolar side chains, the natural or non-natural uncharged D- or L-amino acids with polar side chains, amino acids with aromatic side chains, the natural or non-natural positively charged D- or L- amino acids, the natural or non-natural negatively charged D- or L amino acids as well as within any amino acids of similar size and molecular weight, wherein the molecular weight of the original amino acid should not deviate more than approximately +/- 25% of the molecular weight of the original amino acid and the binding capacity to the receptor of thrombopoietin with agonistic effect is maintained.

The peptide sequences described herein can be used as suitable monomeric peptide units which constitute binding domains for the TPO receptor. They can be used in there monomeric form since they bind to the TPO receptor. As described herein, they are preferably used as dimers since it was shown that the capacity to induce dimerisation of the TPO receptor and thus biological activity is enhanced by dimerisation of the monomeric binding units.

At the beginning (N-terminal) and end (C-terminal) of the described individual peptide consensus sequences, amino acids may be added. It is self-evident that size is not of relevance as long as the peptide function is preserved. Furthermore, please note that individual peptide sequences that might be too short to enfold their activity as monomers usually function as agonists upon dimerisation. Such peptides are thus preferably used in their dimeric form. Respective truncated and or elongated embodiments are thus also comprised by the spirit of the invention.

In the present invention, the abbreviations for the one-letter code as capital letters are those of the standard polypeptide nomenclature, extended by the addition of non-natural amino acids.

| Code | Amino acid |
|---|---|
| A | L-alanine |
| V | L-valine |
| L | L-leucine |
| I | L-isoleucine |
| M | L-methionine |
| F | L-phenylalanine |
| Y | L-tyrosine |
| W | L-tryptophan |
| H | L-histidine |
| S | L-serine |
| T | L-threonine |
| C | L-cysteine |
| N | L-asparagine |
| Q | L-glutamine |
| D | L-aspartic acid |
| E | L-glutamic acid |
| K | L-lysine |
| R | L-arginine |
| P | L-proline |
| G | glycine |
| Ava, 5-Ava | 5-aminovaleric acid |
| Als, 5-Als | 5-aminolevulinic acid |
| MeG | N-methylglycine |
| AcG | N-acetylglycine |
| Hsm | homoserine methylether |
| Har | homoarginine |
| 1nal | 1-naphthylalanine |
| 2nal | 2-naphthylalanine |
| βAla | beta-alanin |
| hoc/hcy | homocysteine |
| Ac | acetylated |
| Am | amidated |
| Dap | diamino propionic acid |
| Dab | diamino butyric acid |
| Aad | alpha-amino adipic acid |
| Asu | alpha-aminosuberic acid |
| Adi | adipic acid, |
| Glr | glutaric acid |
| Sec | selenocysteine |
| Sar | sarcosine |

As described above, the present invention also includes modifications of the peptides and defined peptide consensuses by conservative exchanges of single amino acids. Such exchanges alter the structure and function of a binding molecule but only slightly in most cases. In a conservative exchange, one amino acid is replaced by another amino acid within a group with similar properties.

Examples of corresponding groups are:
- amino acids having non-polar side chains: A, G, V, L, I, P, F, W, M
- uncharged amino acids having polar side chains: S, T, G, C, Y, N, Q
- amino acids having aromatic side chains: F, Y, W
- positively charged amino acids: K, R, H
- negatively charged amino acids: D, E
- amino acids of similar size or molecular weight, wherein the molecular weight of the replacing amino acids deviates by a maximum of +/- 25% (or +/- 20%, +/- 15%, +/- 10%) from the molecular weight of the original amino acid.

It is self evident, that the groups also include non-proteinogenic natural or non-natural amino acids with the respective side chain profile such as e.g. homoarginine in case of the group depicting positively charged side chains.

As outlined, the peptides according to the invention may comprise besides L-amino acids or the stereoisomeric D- amino acids, unnatural/unconventional amino acids, such as e.g. alpha, alpha-disubstituted amino acids, N-alkyl amino acids or lactic acid, e.g. 1-naphthylalanine, 2-naphthylalanine, homoserine-methylether, beta-alanine, 3-pyridylalanine, 4-hydroxyproline, O-phosphoserine, N-methylglycine (sarcosine), homoarginine, N-acetylserine, N-acetylglycine, N-formylmethionine, 3-methylhistidine, 5-hydroxylysine, nor-lysine, 5-aminolevulinic acid or aminovaleric acid. The use of N-methylglycine (MeG) and N-acetylglycine (AcG) is especially preferred, in particular in a terminal position. Also within the scope of the present invention are peptides which are retro, inverso and retro/inverso peptides of the defined peptides and those peptides consisting entirely of D-amino acids.

The present invention also relates to the derivatives of the peptides, e.g. oxidation products of methionine, or deamidated glutamine, arginine and C-terminus amide.

Preferably the described peptides are modified as to AcG at the N-terminus and MeG at the C-terminus.

The peptides and compounds of the present invention have a broad spectrum of utility:

The peptides and compound of the present invention have the ability to bind to and in case of TPO agonistic peptide activate the TPO receptor, and/or have the ability to stimulate the production (both *in vivo* and *in vitro*) of platelets ("thrombopoietic activity") and platelet precursors ("megakaryocytopoietic activity"). To measure the activity (-ies) of these compounds, one can utilize standard assays, such as those described in WO 95/26746 entitled "Compositions and Methods for Stimulating Megakaryocyte Growth and Differentiation" herein incorporated by reference. *In vivo* assays are further described in the state of the art such as for example in WO 00/24770 herein incorporated by reference.

The conditions to be treated by the methods and compositions of the present invention are generally those which involve an existing megakaryocyte/platelet deficiency or an expected or anticipated megakaryocyte/platelet deficiency in the future (e.g., because of planned surgery or platelet donation). Such conditions may be the result of the deficiency (temporary or permanent) of active Mpl ligand *in vivo.* The generic term for platelet deficiency is thrombocytopenia, and hence the methods and compositions of the present invention are generally available for prophylactically or therapeutically treating thrombocytopenia in patients in need thereof.

Thrombocytopenia (platelet deficiencies) may be present for various reasons, including chemotherapy and other therapy with a variety of drugs, radiation therapy, surgery, accidental blood loss, and other specific disease conditions. Exemplary specific disease conditions that involve thrombocytopenia and may be treated in accordance with this invention are: aplastic anemia; idiopathic or immune thrombocytopenia (ITP), including idiopathic thrombocytopenic purpura associated with breast cancer; HIV associated ITP and HIV-related thrombotic thrombocytopenic purpura; metastatic tumors which result in thrombocytopenia; systemic lupus erythematosus; including neonatal lupus syndrome splenomegaly; Fanconi's syndrome; vitamin B12 deficiency; folic acid deficiency; May-Hegglin anomaly; Wiskott-Aldrich syndrome; chronic liver disease; myelodysplastic syndrome associated with thrombocytopenia; paroxysmal nocturnal hemoglobinuria; acute profound thrombocytopenia following C7E3 Fab (Abciximab) therapy; alloimmune thrombocytopenia, including maternal alloimmune thrombocytopenia; thrombocytopenia associated with antiphospholipid antibodies and thrombosis; autoimmune thrombocytopenia; drug-induced immune thrombocytopenia, including carboplatin-induced thrombocytopenia, heparin-induced thrombocytopenia; fetal thrombocytopenia; gestational thrombocytopenia; Hughes' syndrome; lupoid thrombocytopenia; accidental and/or massive blood loss; myeloproliferative disorders; thrombocytopenia in patients with malignancies; thrombotic thrombocytopenia purpura, including thrombotic microangiopathy manifesting as thrombotic thrombocytopenic purpura/hemolytic uremic syndrome in cancer patients; autoimmune hemolytic anemia; occult jejunal diverticulum perforation; pure red cell aplasia; autoimmune thrombocytopenia; nephropathia epidemica; rifampicin-accociated acute renal failure; Paris-Trousseau thrombocytopenia; neonatal alloimmune thrombocytopenia; paroxysmal nocturnal hemoglobinuria; hematologic changes in stomach cancer; hemolytic uremic syndromes in childhood; hematologic manifestations related to viral infection including hepatitis A virus and CMV-associated thrombocytopenia. Also, certain treatments for AIDS result in thrombocytopenia (for example AZT). Certain wound healing disorders might also benefit from an increase in platelet numbers.

The compounds of this invention may also be useful in stimulating certain cell types other than megakaryocytes if such cells are found to express the TPO receptor. Conditions associated with such cells that express the TPO receptor, which are responsive to stimulation by the peptides of the present invention are also within the scope of the present invention.

The compounds of this invention may thus be used in any situation in which production of platelets or platelet precursor cells is desired, or in which stimulation of the TPO receptor is desired. Thus, for example, the compounds of this invention may be used to treat any condition in a mammal wherein there is a need of platelets, megakaryocytes, and the like. Such conditions are described in detail for example in WO 00/24770 herein incorporated by reference (also see above).

The compounds of the present invention have strong binding properties to the TPO receptor. TPO mimetic peptides act as agonists and can thus activate the TPO receptor. Accordingly, such compounds are useful for therapeutic purposes in treating conditions which can be ameliorated by stimulation of TPO receptors, for example on platelets, megakaryocytes and other stem cells, for example thrombocytopenia which may be as a result of, for example, chemotherapy, radiation therapy, autologous, allogeneic and syngeneic bone marrow transplantation, peripheral blood progenitor cell and cord blood progenitor cell transplantation, biological or other treatments for malignant and non-malignant disease (for example interferon and other cytokines), bone marrow infiltration by metastatic tumours and leukaemia, bone marrow infiltration or encroachment in non-malignant diseases (for example osteopetrosis, Gaucher's disease), myelodysplastic syndrome, myelofibrosis, and related syndromes (both primary and secondary), immune and idiopathic disease, including autoimmune and alloimmune thrombocytopenias, and drug-induced immune thrombocytopenia, drug-induced thrombocytopenia (for example due to anti-retroviral therapy in HIV-positive patients), chronic liver diseases and chronic renal failure, excessive platelet destruction, including thrombotic thrombocytopenic purpura, haemolytic uraemic syndrome and other microangiopathies, and hypersplenism, congenital syndromes where bleeding due to deficient or abnormal platelets occurs for example Glanzmann's thrombasthenia, thrombocytopenia with absent radius (TAR) syndrome, gray platelet syndrome, liver transplantation, repair of aortic aneurysms, intra-aortic balloon counterpulsation, coronary artery bypass grafting and other surgical procedures requiring extracorporeal circulation and/or massive blood transfusion, or HIV-related thrombocytopenia; also granulocytopenia and anaemia which may be a result of, for example, chemotherapy, radiotherapy and other therapy for malignant and non-malignant diseases (as discussed above), bone marrow infiltration in malignant, pre-malignant and non-malignant diseases (as discussed above), immune and non-immune, idiopathic and drug-related anaemia and granulocytopenia; or the treatment of haematological malignancies *per se*. The compounds according to the invention may also be used for therapeutic, diagnostic and research purposes where stimulation (in case of TPO mimetics) or binding of TPO receptors, for example on platelets, megakaryocytes and other stem cells, is desirable for example in the mobilisation of peripheral blood progenitor cells for autologous, allogeneic or syngeneic transplantation, increasing the yield from platelet and other blood donors, prolongation of platelet survival and quality *ex vivo,* production or expansion of platelets and/or progenitor cells *in vitro;* for diagnostic purposes in studying the mechanism of hematopoiesis and for the *in vitro* expansion of megakaryocytes and committed progenitor cells.

As discussed hereinabove the compounds according to the invention are useful for the prevention and treatment of diseases mediated by TPO, for example haematological disorders including thrombocytopenia, granulocytopenia and anaemia, and the treatment of haematological malignancies. Thus, the present invention also provides a method for treating a patient suffering from a disorder that is susceptible to treatment with a thrombopoietin agonist comprising administering to the patient a therapeutically effective dose or amount of a compound of the present invention.

The invention further provides a compound according to the invention for use in therapy, in particular in human medicine.

There is also provided as a further aspect of the invention the use of a compound according to the invention in the preparation of a medicament for use in the treatment of conditions mediated by thrombopoietin agonists.

It will be appreciated that reference to treatment is intended to include prophylaxis as well as the alleviation of established symptoms.

The invention also provides for pharmaceutical compositions comprising one or more of the compounds described herein and a physiologically acceptable carrier. These pharmaceutical compositions can be in a variety of forms including oral dosage forms, as well as inhalable powders and solutions and injectable and infusible solutions.

### Figures

- Fig. 1: shows a table extracted from the state of the art (Dower et al., 1998) depicting the family classification of the TPO mimetic peptides known in the state of the art.
- Fig. 2: shows that the TPO mimetic peptides known in the state of the art do not bind to other receptors of the cytokine family, such as especially the EPO receptor. This indicates that TPO mimetic peptides and EPO mimetic peptides even though showing some similarities are in fact structurally different.
- Fig. 3: shows an example of a simple supravalent molecule according to the present invention. Two dimeric TPO mimetic peptides are connected N-terminally by a bifunctional PEG moiety carrying maleimide groups.
- Fig. 4: shows an example using HES as polymeric carrier unit. HES was modified such that it carries maleimide groups reacting with the SH-groups of the peptide units.
- Fig. 5: shows an example of a biodegradable polymeric carrier structure.

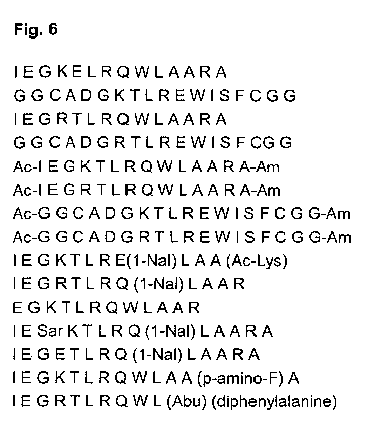

- Fig. 6: discloses several TPO mimetic peptides according to the present invention which carry a charged amino acid in position X₆.

### Examples/Production

### Preparation of peptides

The peptides of the invention can be prepared by classical methods known in the art, for example, by using standard solid phase techniques, see, for example, Merrifield, J. Am. Chem. Soc, 85:2149 (1963), incorporated herein by reference. The standard methods include exclusive solid phase synthesis, partial solid phase synthesis methods, fragment condensation, classical solution synthesis, and even recombinant DNA technology. On solid phase, the synthesis is typically commenced from the C-terminal end of the peptide using an alpha-amino protected resin. A suitable starting material can be prepared, for instance, by attaching the required alpha-amino-acid to a chloroemethylated resin, a hydroxymethyl resin, or a benzhydrylamine resin.

According to one embodiment used for the present examples the synthesis is carried out by the use of a Liberty microwave system (CEM) using Rink-Amide-Resin (substitution rate 0.19mmol/g) in a scale of 0.25mmol. Removal of Fmoc-groups is achieved by double treatment with 10ml piperidine/DMF (1:3) and irridation with 50W for 200sec. Coupling of amino acids is achieved by double treatment with a of 4fold excess of amino acid in DMF PyBOP/HOBT/DIPEA as coupling additives and irradiation with 50W for 300sec. Between all irridation cycles the solution is cooled by bubbling nitrogen through the reaction mixture. After deprotection and coupling, the resin is washed 6 times with 10ml DMF. After the double coupling cycle all unreacted amino groups are blocked by treatment with a 10fold excess of N-(2-Chlorobenzyloxycarbonyloxy) succinimide (0.2M solution in DMF) and irridation with 50W for 120sec. After deprotection of the last amino acid, the peptide is acetylated by incubation with 0.793ml of capping-solution (4.73ml acetic anhydride and 8.73ml DIEA in 100ml DMSO) for 5 minutes. Before cleavage the resin is then washed 6 times with 10ml DMF and 6 times with 10ml DCM. Cleavage of the crude peptides is achieved by treatment with 20ml TFA/TIS/EDT/H₂O (94/1/2.5/2.5) for 120 minutes under an inert atmosphere. This solution is filtered into 80ml cold ether, the precipitate dissolved in acetonitrile / water (1/1) and the peptide is purified by RP-HPLC (Kromasil 100 C18 10µm, 250x4.6mm).

### Cyclisation of the peptides

### Cyclization with K₃[(FeCN₆)

Solution1: 10mg of the peptide are dissolved in 0.1% TFA/acetonitrile and diluted with water until a concentration of 0.5mg/ml is reached. Solid ammonium bicarbonate is added to reach a pH of app. 8.
Solution 2: In a second vial 10ml 0.1 % TFA/acetonitrile are diluted with 10ml of water. Solid ammonium bicarbonate is added until a pH of 8 is reached and 1 drop of a 0.1M solution of K₃[(FeCN₆)] is added.
Solution 1 and 2 are added dropwise over a period of 3 hours to a mixture of acetonitrile/water (1/1; pH = 8). The mixture is incubated at room temperature overnight and the mixture concentrated and purified by LCMS.

### Cyclization with CLEAR-OX™-resin

To 100ml of acetonitrile/water (1/1; 0.1% TFA), solid ammonium bicarbonate is added until a pH of 8 is reached. This solution is degassed by bubbling Argon for 30 minutes. Now 100mg of CLEAR-OX™-resin is added. After 10 minutes, 10mg of the peptide is added as a solid. After 2h of incubation, the solution is filtered, concentrated and purified by LCMS.

### Purification of cyclic peptides:

All peptides were purified using a Nebula-LCMS-system (Gilson). The crude material of all peptides was dissolved in acetonitrile/water (1/1) or DMSO and the peptide was purified by RP-HPLC (Kromasil 100 C18 or C8 10µm, 250x4.6mm). The flow rate was 20ml/min and the LCMS split ratio 1/1000.

### TPO-in vitro Assay - Proliferation assay M07e cells by MTT

The following protocol allows measuring the TPO activity *in vitro:*
Day 1: Preparation (starvation) of the M07e cells
   • Use M07e cells in logarithmic growth phase (4.10⁵ - 2.10⁶ cells/ml) grown in RPMI complete (20% fetal calf serum - FCS) with 10 ng/ml IL-3. Count cells.
   • Take the amount of cells needed for the assay from flask, centrifuge 5 min. 1500 rpm and resuspend in RPMI with 20 % FCS and without IL3 at 600.000 cells/ml. Preculture without IL-3 for 72 hours. Count cells again before starting the assay
Day 3 or 4: Addition of different TPO-concentrations
   • Use 96-well plates to test the efficacy of peptides or TPO in 18 concentrations, each concentration in quadruplicate. Leave the uppermost and lowermost line of wells free
   • Prepare stock solutions of the reagents to be tested freshly in RPMI with 20 % FCS: for peptides the stock solutions are usually 1 mg/ml. Prepare a dilution scheme: highest concentration (stock solution) in the leftmost well of the second line, dilute the reagent into the fifth and the ninth well of this line, and continue diluting into the next line of wells
      -for preparing 1:√10 dilutions: start with 292 µl stock solution - >pipette 92 µl into next well containing 200µl RPMI with 20% FCS -> mix and pipette 92 µl into next well, etc.)
      Controls: always perform the assay with one plate containing different concentrations of recombinant TPO: TPO stock solution is 1 µg/ml.

The concentration of agents during the assay will be half of that directly after preparing the dilutions
• Pipette 3 x 50 µl volumes from the first, fifth and ninth wells of each line into the next three wells, so that every concentration of agent is represented 4 times
• Take cells needed for the entire assay from culture flask, centrifuge 5 min. 1500 rpm, resuspend in RPMI with 20% FCS at 1 000 000 cells/ml
• Add 50 µl (50.000 cells) of cell suspension to each well
• Incubate at 37°C for 3 days

### Day 5-7: MTT-Assay

• Before performing MTT assay, prepare a dilution range of known amounts of TF-1 cells into wells: 0/10000/25000/50000/100000/250000 cells/well in 100 µl medium
• Thaw ready-to-use MTT reagent (Promega, CellTiter 96 Aqueous One Solution Cell Proliferation Assay) in 37°C waterbath
• Add 20 µl of MTT solution to each well
• Incubate 1.5-2 hours at 37°C. Shortly shake plate to evenly distribute dye
• Add 25 µl of 10% SDS to each well to stop reaction
• Measure E492 nm (reference 620 nm) in ELISA reader

### References:

Kato T, Matsumoto A, Ogami K, Tahara T, Morita H, Miyazaki H: "Native Thrombopoietin: Structure and Function." STEM CELLS 1998; 16:322-328.
Jagerschmidt A, Fleury V, Anger-Leroy M, Thomas C, Agnel M, O'Brien DP: "Human thrombopoietin structure-function relationships: identification of functionally important residues." Biochem. J. (1998) 333, 729-734.
Cytokynes, 2004; page 975.
Orita T, Tsunoda H, Yabuta N, Nakano K, Yoshino T, Hirata Y, Ohtomo T, Nezu J. Sakumoto H, Ono K, Saito M, Kumagai E, Nanami M, Kaneko A, Yoshikubo T: "A novel therapeutic approach for thrombocytopenia by minibody agonist of the thrombopoietin receptor." BLOOD, 15 January 2005, VOLUME 105, no. 2, 562-566.
Dower WJ, Cwirla SE, Balasubramanian P, Schatz PJ, Baccanari DP, Barrett W: "Peptide Agonists of the Thrombopoietin Receptor." STEM CELLS 1998; 16(suppl 2):21-29.
Cwirla SE, Balasubramanian P, Duffin DJ, Wagstrom CR, Gates CM, Singer SC, Davis AM, Tansik RL, Mattheakis LC, Boytos CM, Schatz PJ, Baccanari DP, Wrighton NC, Barrett RW, Dower WJ: "Peptide Agonist of the Thrombopoietin Receptor as Potent as the Natural Cytokine." SCIENCE, VOL. 276, 13 June 1997, 1696-1699.
Kimura T, Kaburaki H, Miyamoto S, Katayama J, Watanabe Y: "Discovery of a Novel Thrombopoietin Mimic Agonist Peptide." J. Biochem. 122, 1046-1051 (1997).
Wrighton NC, Balasubramanian P, Barbone FP, Kashyap AK, Farrell FX, Jolliffe L, Barrett RW, Dower WJ (1997) Increased potency of an erythropoietin peptide mimetic through covalend dimerization. Nature Biotechnology 15:1261-1265.
De Serres M, Ellis B, Dillberger JE, Rudolph SK, Hutchins JT, Boytos CM, Weigl DL, DePrince RB: "Immunogenicity of Thrombopoietin Mimetic Peptide GW395058 in BALB/c Mice and New Zealand White Rabbits: Evaluation of the Potential for Thrombopoietin Neutralizing Antibody Production in Man." STEM CELLS 1999; 17:203-209.
De Serres M, Yeager RL, Dillberger JE, Lalonde G, Gardner GH, Rubens CA, Simkins AH, Sailstad JM, McNulty MJ, Woolley JL: "Pharmacokinetics and Hematological Effects of the PEGylated Thrombopoietin Peptide Mimetic GW395058 in Rats and Monkeys After Intravenous or Subcutaneous Administration." STEM CELLS 1999; 17:316-326.
Case BC, Hauck ML, Russell YL, Simkins AH, de Serres M, Schmith VD, Dillberger JE, Page RL: "The Pharmacokinetics and Pharmacodynamics of GW395058, a Peptide Agonist of the Thrombopoietin Receptor, in the Dog, a Large-Animal Model of Chemotherapy-Induced Thrombocytopenia." STEM CELLS 2000;18:360-365.
Roberts, M. J., M. D. Bentley, et al. (2002). "Chemistry for peptide and protein PEGylation." Advanced Drug Delivery Review 54(4): 459-476.
Zhao, X. et al (1997), "Novel Degradable Poly(ethylene glycol) esters for drug delivery." In "Poly(ethylene glycol) chemistry and biological applications; Harris JM, Zalipsky, S. Eds.; ACS Symposium Series 680; American Chemical Society: Washington DC, 1997; 458-472.
Richard Tacey, Anthony Greway, Janice Smiell, David Power, Arno Kromminga, Mohamed Daha, Nicole Casadevall and Marian Kelley: The detection of anti-erythropoietin antibodies in human serum and plasma - Part 1. Validation of the protocol for a radioimmunoprecipitation assay; J Immunol Methods. 2003 Dec;283(1-2):317-29. Zalipsky S, Qazen, S, Walker II JA, Mullah N, Quinn YP, (1999) "New detachable poly (ethylene glycol) conjugates: Cysteine-cleavable lipopolymers regenerating natural phospholipid, diacyl phosphatidylethanolamine, Bioconjug. Chem. 10: 703-707.
Haag R, Sunder A, Stumbé JF, J. Am. Chem. Soc. (2000), 122, 2954.
Merrifield, J. Am. Chem. Soc, 85:2149 (1963)

## Claims

1. A peptide, especially one capable of binding the TPO receptor, selected from the group consisting of
- peptides comprising the following consensus sequence of amino acids:
X₅X₆X₇X₈X₉X₁₀X₁₁X₁₂
wherein each amino acid is selected from natural or unnatural amino acids and
X₅ is G, A, a conservative exchange of G or Sar,
X₆ is a charged amino acid,
X₇ is T, F, K, L, N, Q, R, S or V,
X₈ is L, C, F, I, M, R, S, V, W or Sec
X₉ is selected from any amino acid,
X₁₀ is Q, A, D, E, G, K, M, R, S, T, V, Y or N,
X₁₁ is W, C, F, G, L, M, S, Y, 1-Nal, 2-Nal, A, L-(benzothienyl)-alanine, 5-MeW, 1-Me-W or Sec,
X₁₂ is L, C, G, I, K, M, N, R, V, A, F or Sec and
- functionally equivalent fragments, derivatives and variants of the peptides defined by the above consensus sequence, that bind the TPO receptor and have a charged amino acid in position X₆.

2. A peptide according to claim 1, wherein said sequence of amino acids is dimerised in order to provide a peptide having two TPO receptor binding moieties.

3. A peptide according to claims 1 or 2, comprising additionally at least one of the following amino acid positions:
X₁X₂X₃X₄
wherein said amino acids positions are selected from any amino acid, however are preferably chosen as follows:
X₁ is L, G an amino acid depicting a side chain functionality capable of forming a covalent bond, especially C,
X₂ is C, Sec, H, Acyl, A, N, G, T, I or an amino acid depicting a side chain functionality capable of forming a covalent bond,
X₃ is A, C, Sec, E, G, I, L, M, P, R, Q, S, T or V,
X₄ is A, C, Sec, D, E, K, L, Q, R, S, T, V or G.

4. A peptide according to anyone of claims 1 to 3 comprising additionally at least one of the following amino acid positions:
X₁₃X₁₄X₁₅X₁₆X₁₇X₁₈X₁₉
wherein X₁₃ to X₁₉ are selected from any amino acid but are preferably defined as follows:
X₁₃ is L, H, S, A, Ava, Abu, I, V, L, F, T, K or E,
X₁₄ is G, F, A, diphenylalanine, I, V, L, S, Q, C or an amino acid depicting a side chain functionality capable of forming a covalent bond,
X₁₅ is G, C, Sec, N, R, p-amino phenylalanine Ac-K, R, Ava, K, T, V, Q or an amino acid depicting a side chain functionality capable of forming a covalent bond,
X₁₆ is F, G, A, beta-A, N-methyl-A, Sar, I, V, L, T, R or E,
X₁₇ is C, G, beta-A, K, D or R,
X₁₈ is G, K, beta-A, D, N, R or A,
X₁₉ is G, P, T, N or R.

5. A peptide according to at least one of the claims 1 to 4 wherein said sequence of amino acid is cyclised.

6. A peptide according to claim 5 wherein cyclisation is achieved between two amino acids depicting a side chain functionality capable of forming a covalent bond.

7. A peptide according to claim 5 or 6 wherein the amino acids responsible for cyclisation are chosen such that they are capable of forming an intramolecular bridge within the peptide by forming a covalent bond between their side chains.

8. A peptide according to at least one of the claims 5 to 7 wherein said bridge is either a disulfide or a diselenide bridge.

9. A peptide according to at least one of the claims 5 to 8 wherein the amino acids involved in cyclisation are selected from the group comprising cysteine, cysteine derivatives such as homocysteine and selenocysteine, thiolysine, K, R, D or E.

10. A peptide according to at least one of the claims 5 to 9, wherein said covalent bond for cyclisation is formed between two amino acids that are separated by 12 amino acids.

11. A peptide according to at least one of the claims 5 to 10, having the following structure:
CA(AS₁₋₂)X₄-X₁₂(AS₁₋₂)CA
wherein CA represents an amino acid involved in forming a covalent bond for cyclisation of the peptide and AS₁₋₂ means that there may be one or two amino acids present.

12. A peptide according to at least one of the above claims 1 to 11 wherein the amino acid positions if present in the peptide consensus are defined as follows:
X₁ is an amino acid depicting a side chain functionality capable of forming a covalent bond, preferably C,
X₂ is preferably an amino acid depicting a side chain functionality capable of forming a covalent bond, in case X₁ is not an amino acid depicting a side chain functionality capable of forming a covalent bond,
X₃ is alanine,
X₄ is D, E or K,
X₅ is glycine,
X₆ is either a negatively or a positively charged amino acid such as K, R, H, D, E, homoarginine or alpha-amino adipic acid,
X₇ is F, R, S or T,
X₈ is F, L, V or W,
X₉ is A, K, L, M, R, S, V, T, E, G, H, P, Q, W, Nle, Ac-K, more preferably A, K, L, M, R, S, V or T and most preferred R.
X₁₀ is E, G, K, M, Q, R, S or T, more preferably E or Q,
X₁₁ is W, F, L, A, 1-Nal or 2-Nal, L-(benzothienyl)-alanine, 5-Me-W or 1-Me-W,
X₁₂ is C, I, K, L, M or V, more preferably I, K, L, R or V,
X₁₄ is preferably an amino acid depicting a side chain functionality capable of forming a covalent bond in case X₁₅ is not an amino acid depicting a side chain functionality capable of forming a covalent bond,
X₁₅ is preferably an amino acid depicting a side chain functionality capable of forming a covalent bond in case X₁₄ is not an amino acid depicting a side chain functionality capable of forming a covalent bond.

13. A TPO mimetic peptide, comprising at least two peptide consensus sequences defining a monomeric TPO receptor binding unit, wherein at least one of the monomeric peptide consensus sequences comprises an amino acid consensus sequence according to at least one of the claims 1 to 12.

14. A peptide according to at least one of the claims 1 to 13, wherein at least one carrier unit is coupled to the peptide, wherein said carrier is or comprises at least one natural or synthetic branched, dendritic or linear polymer and is preferably selected from the group consisting of polyglycerines, polysialic acid, dextrans, starches or polyethylenglycol or from other biologically inert water soluble polymers or is a Fc region.

15. A compound binding the TPO receptor, comprising
i) at least two peptide units wherein each peptide unit comprises at least two domains with a binding capacity to the TPO receptor;
ii) at least one polymeric carrier unit;
wherein said peptide units are attached to said polymeric carrier unit and wherein at least one domain of at least one peptide unit comprises an amino acid sequence according to at least one of the claims 1 to 12.

16. A compound according to claim 15 wherein at least one of the peptide units comprises a peptide dimer according to claim 14.

17. A compound according to at least one of the claims 15 or 16, wherein said carrier unit is or comprises at least one natural or synthetic branched, dendritic or linear polymer and is preferably selected from the group consisting of polyglycerines, polysialic acid, dextrans, starches or polyethylenglycol or from other biologically inert water soluble polymers.

18. A compound according to at least one of the claims 15 to 17, wherein said peptide unit is attached via a covalent bond to said polymeric carrier unit and attachment occurs via a reactive amino acid, the N-terminal amino group and/or the C-terminal carboxylic acid of said peptide units, wherein said reactive amino acid is preferably selected from the group consisting of lysine, cysteine, histidine, arginine, aspartic acid, glutamic acid, serine, threonine and tyrosine and
- wherein in case said polymeric carrier unit does not possess an appropriate reactive coupling group, a coupling unit is used for modifying the polymeric carrier unit,
- wherein said coupling unit is preferably selected from the group consisting of acylating groups which react with the amino groups of said peptide unit, alkylating groups which react with sulfhydryl (mercapto), thiomethyl, imidazo or amino groups on said peptide unit, most preferably maleimide groups, ester and amide forming groups which react with a carboxyl group of the protein, disulfide forming groups which react with the sulfhydryl groups on said peptide unit, such as 5,5'-dithiobis (2-nitrobenzoate) groups, ortho-pyridyl disulfides and alkyl mercaptan groups, dicarbonyl groups, such as cyclohexandione groups, and other 1,2-diketone groups which react with the guanidine moieties of said peptide unit; diazo groups, which react with phenolic groups on said peptide; reactive groups from reaction of cyanogens bromide with said polymeric carrier unit, which react with amino groups on said peptide unit.

19. A method for dimerising monomeric peptide units comprising an amino acid consensus sequence as defined in at least one of the claims 1 to 12 to form an TPO mimetic peptide dimer, wherein the dimer is created by forming a covalent bond between the monomeric peptide units, wherein said bond is formed between the C-terminal amino acid of the first monomeric peptide unit and the N-terminal amino acid of the second monomeric peptide unit.

20. A method according to claim 19, wherein monomeric peptide units are used, carrying an amino acid at either the C- or the N-terminus for the side chain functionality capable of forming a covalent bond, wherein a covalent bond is formed between the side chain of the C-terminal amino acid of the first monomeric peptide unit and the side chain of the N-terminal amino acid of the second monomeric peptide unit.

21. A pharmaceutical composition comprising a peptide or a compound according to at least one of the claims 1 to 18 in a therapeutically effective amount and optionally a pharmaceutically acceptable excipient.

22. Use of a peptide or a compound according to at least one of the claims 1 to 18 for the manufacture of a medicament for treating a patient suffering from a disorder that is susceptible to treatment with a thrombopoietin agonist.

23. A method for treating a patient suffering from a disorder that is susceptible to treatment with a thrombopoietin agonist, comprising administering to the patient, a therapeutically effective dose or amount of a peptide or a compound according to at least one of the claims 1 to 18 or 21.

24. The method of claim 23, wherein the disorder susceptible to treatment with a thrombopoietin agonist is selected from the group consisting of haematological disorders and thrombocytopenia resulting from chemotherapy, radiation therapy, or bone marrow transfusions.


SEQUENCE LISTING

<110>  AplaGen GmbH

<120>  Peptides binding the TPO receptor

<130>  47510

<140>  EP06018604.6
<141>  2006-09-05

<160>  18

<170>  PatentIn version 3.3

<210>  1
<211>  8
<212>  PRT
<213>  Artificial

<220>
<223>  TPO mimetic peptides


<220>
<221>  MISC_FEATURE
<222>  (1)..(1)
<223>  X5 is G, A, a conservative exchange of G or Sar

<220>
<221>  MISC_FEATURE
<222>  (2)..(2)
<223>  X6 is a charged amino acid

<220>
<221>  MISC_FEATURE
<222>  (3)..(3)
<223>  X7 is T, F, K, L, N, Q, R, S or V

<220>
<221>  MISC_FEATURE
<222>  (4)..(4)
<223>  X8 is L, C, F, I, M, R, S, V, W or Sec

<220>
<221>  MISC_FEATURE
<222>  (5)..(5)
<223>  X9 is selected from any amino acid

<220>
<221>  MISC_FEATURE
<222>  (6)..(6)
<223>  X10 is Q, A, D, E, G, K, M, R, S, T, V, Y or N

<220>
<221>  MISC_FEATURE
<222>  (7)..(7)
<223>  X11 is W, C, F, G, L, M, S, Y, 1-Nal, 2-Nal, A,
       L-(benzothienyl)-alanine or 5-Me-W, 1-Me-W, or Sec

<220>
<221>  MISC_FEATURE
<222>  (8)..(8)
<223>  X12 is L, C, G, I, K, M, N, R, V, A, F or Sec

<400>  1

Xaa Xaa Xaa Xaa Xaa Xaa Xaa Xaa

1                   5


<210>  2
<211>  4
<212>  PRT
<213>  Artificial

<220>
<223>  TPO mimetic peptides


<220>
<221>  MISC_FEATURE
<222>  (1)..(1)
<223>  X1=any natural or unnatural amino acid, preferaby L, G, or an
       amino acid depicting a side chain functionality capable of
       forming a covalent bond such as for example cysteine,
       homocysteine or selenocysteine

<220>
<221>  MISC_FEATURE
<222>  (2)..(2)
<223>  X2=any natural or unnatural amino acid, preferaby H, A, N, G, T,
       I or an amino acid depicting a reactive side chain functionality
       capable of forming a covalent bond such as for example cysteine,
       homocysteine or selenocysteine

<220>
<221>  MISC_FEATURE
<222>  (3)..(3)
<223>  X3=any natural or unnatural amino acid, preferaby A, C, E, G, I,
       L, M, P, R, Q, S, T or V

<220>
<221>  MISC_FEATURE
<222>  (4)..(4)
<223>  X4=any natural or unnatural amino acid, preferaby A, C, D, E, K,
       L, Q, R, S, T, V or G

<400>  2

Xaa Xaa Xaa Xaa
1


<210>  3
<211>  7
<212>  PRT
<213>  Artificial

<220>
<223>  TPO mimetic peptide


<220>
<221>  MISC_FEATURE
<222>  (1)..(1)
<223>  X13 is any natural or unnatural amino acid, preferably L, H, S,
       A, Ava, Abu, I, V, L, F, T, K or E

<220>
<221>  MISC_FEATURE
<222>  (2)..(2)
<223>  X14 is any natural or unnatural amino acid, preferably G, F, A,
       diphenylalanine, I, V, L, S, Q, C or an amino acid depicting a
       side chain functionality capable of forming a covalent bond

<220>
<221>  MISC_FEATURE
<222>  (3)..(3)
<223>  X15 is any natural or unnatural amino acid, preferably G, C,
       selenocysteine, N, R, p-amino phenylalanine, Ac-K, R, Ava, K, T,
       V, Q or an amino acid depicting a side chain functionality
       capable of forming a covalent bond

<220>
<221>  MISC_FEATURE
<222>  (4)..(4)
<223>  X16 is any natural or unnatural amino acid, preferably F, G, A,
       beta-A, N-methyl-A, Sar, I, V, L, T, R or E

<220>
<221>  MISC_FEATURE
<222>  (5)..(5)
<223>  X17 is any natural or unnatural amino acid, preferably C, G,
       beta-A, K, D or R

<220>
<221>  MISC_FEATURE
<222>  (6)..(6)
<223>  X18 is any natural or unnatural amino acid, preferably G, K,
       beta-A, D, N, R or A

<220>
<221>  MISC_FEATURE
<222>  (7)..(7)
<223>  X19 is any natural or unnatural amino acid, preferably G, P, T, N
       or R

<400>  3

Xaa Xaa Xaa Xaa Xaa Xaa Xaa
1               5


<210>  4
<211>  14
<212>  PRT
<213>  Artificial

<220>
<223>  TPO mimetic peptide

<400>  4

Ile Glu Gly Lys Glu Leu Arg Gln Trp Leu Ala Ala Arg Ala
1               5                   10


<210>  5
<211>  18
<212>  PRT
<213>  Artificial

<220>
<223>  TPO mimetic peptide

<400>  5

Gly Gly Cys Ala Asp Gly Lys Thr Leu Arg Glu Trp Ile Ser Phe Cys
1               5                   10                  15


Gly Gly

<210>  6
<211>  14
<212>  PRT
<213>  Artificial

<220>
<223>  TPO mimetic peptide

<400>  6

Ile Glu Gly Arg Thr Leu Arg Gln Trp Leu Ala Ala Arg Ala
1               5                   10


<210>  7
<211>  18
<212>  PRT
<213>  Artificial

<220>
<223>  TPO mimetic peptide

<400>  7

Gly Gly Cys Ala Asp Gly Arg Thr Leu Arg Glu Trp Ile Ser Phe Cys
1               5                   10                  15


Gly Gly


<210>  8
<211>  14
<212>  PRT
<213>  Artificial

<220>
<223>  TPO mimetic peptide


<220>
<221>  MOD_RES
<222>  (1)..(1)
<223>  ACETYLATION

<220>
<221>  MOD_RES
<222>  (14)..(14)
<223>  AMIDATION

<400>  8

Ile Glu Gly Lys Thr Leu Arg Gln Trp Leu Ala Ala Arg Ala
1               5                   10


<210>  9
<211>  14
<212>  PRT
<213>  Artificial

<220>
<223>  TPO mimetic peptide

<220>
<221>  MOD_RES
<222>  (1)..(1)
<223>  ACETYLATION

<220>
<221>  MOD_RES
<222>  (14)..(14)
<223>  AMIDATION

<400>  9

Ile Glu Gly Arg Thr Leu Arg Gln Trp Leu Ala Ala Arg Ala
1               5                   10


<210>  10
<211>  18
<212>  PRT
<213>  Artificial

<220>
<223>  TPO mimetic peptide


<220>
<221>  MOD_RES
<222>  (1)..(1)
<223>  ACETYLATION

<220>
<221>  MOD_RES
<222>  (18)..(18)
<223>  AMIDATION

<400>  10

Gly Gly Cys Ala Asp Gly Lys Thr Leu Arg Glu Trp Ile Ser Phe Cys
1               5                   10                  15


Gly Gly



<210>  11
<211>  18
<212>  PRT
<213>  Artificial

<220>
<223>  TPO mimetic peptide


<220>
<221>  MOD_RES
<222>  (1)..(1)
<223>  ACETYLATION

<220>
<221>  MOD_RES
<222>  (18)..(18)
<223>  AMIDATION

<400>  11

Gly Gly Cys Ala Asp Gly Arg Thr Leu Arg Glu Trp Ile Ser Phe Cys
1               5                   10                  15

Gly Gly

<210> 12
<211> 13
<212> PRT
<213> Artificial

<220>
<223> TPO mimetic peptide

<220>
<221> MISC_FEATURE
<222> (9)..(9)
<223> 1-napthylalanine

<220>
<221> MISC_FEATURE
<222> (13)..(13)
<223> acetyl-lysine

<400> 12

Ile Glu Gly Lys Thr Leu Arg Glu Xaa Leu Ala Ala Xaa
1               5                   10

<210> 13
<211> 13
<212> PRT
<213> Artificial

<220>
<223> TPO mimetic peptide

<220>
<221> MISC_FEATURE
<222> (9)..(9)
<223> 1-napthylalanine

<400> 13

Ile Glu Gly Arg Thr Leu Arg Gln Xaa Leu Ala Ala Arg
1               5                   10

<210> 14
<211> 12
<212> PRT
<213> Artificial

<220>
<223> TPO mimetic peptide

<400> 14

Glu Gly Lys Thr Leu Arg Gln Trp Leu Ala Ala Arg
1               5                   10

<210> 15
<211> 14
<212> PRT

<213>  Artificial

<220>
<223>  TPO mimetic peptide


<220>
<221>  MISC_FEATURE
<222>  (3)..(3)
<223>  sarcosine

<220>
<221>  MISC_FEATURE
<222>  (9)..(9)
<223>  1-napthylalanine

<400>  15

Ile Glu Xaa Lys Thr Leu Arg Gln Xaa Leu Ala Ala Arg Ala
1               5                   10


<210>  16
<211>  14
<212>  PRT
<213>  Artificial

<220>
<223>  TPO mimetic peptide


<220>
<221>  MISC_FEATURE
<222>  (9)..(9)
<223>  1-napthylalanine

<400>  16

Ile Glu Gly Glu Thr Leu Arg Gln Xaa Leu Ala Ala Arg Ala
1               5                   10


<210>  17
<211>  14
<212>  PRT
<213>  Artificial

<220>
<223>  TPO mimetic peptide


<220>
<221>  MISC_FEATURE
<222>  (13)..(13)
<223>  p-amino-phenylalanine

<400>  17

Ile Glu Gly Lys Thr Leu Arg Gln Trp Leu Ala Ala Xaa Ala
1               5                   10


<210>  18
<211>  12
<212>  PRT
<213>  Artificial

<220>

<223>  TPO mimetic peptide

<220>
<221>  MISC_FEATURE
<222>  (11)..(11)
<223>  Abu

<220>
<221>  MISC_FEATURE
<222>  (12)..(12)
<223>  Diphenylalanine

<400>  18

Ile Glu Gly Arg Thr Leu Arg Gln Trp Leu Xaa Xaa
1               5                   10